# EUROPEAN PATENT APPLICATION

(11) **EP 4 659 693 A1**
(43) Date of publication of application: **10.12.2025**
(21) Application number: 25180860.6
(22) Date of filing: 04.06.2025
(51) Int. Cl.: A61B 18/12, A61B 18/00

(54) **GENERATOR FOR CONTROLLING AN ELECTROSURGICAL INSTRUMENT**

(30) Priority: 07.06.2024 US 202463657454 P
(71) Applicant: Olympus Winter & Ibe GmbH, 22045 Hamburg (DE)
(72) Inventor: Janich, Fabian, 14469 Potsdam (DE); Ramin, Daniel, 14558 Nuthetal (DE); Knopf, Christoph, 23617 Stockelsdorf (DE); Engels, Oliver, 14169 Berlin (DE)
(74) Representative: Eisenführ Speiser

(57) **Abstract**

Generator for controlling an electrosurgical instrument, in particular a resectoscope in transurethral resection, for cutting, vaporizing or thermally treating body tissues, wherein the electrosurgical instrument has one or two instrument poles, and the electrosurgical instrument is controlled by an RF signal in order to control an electrical instrument current via one instrument pole or via the two instrument poles when the RF signal is activated, wherein the generator is prepared to identify a contact of one or both instrument poles with an electrically conductive solid.

## Description

The present invention is directed to a method for controlling an electrosurgical instrument, in particular a resectoscope in transurethral resection, for cutting, vaporizing or thermally treating body tissues. The invention is also directed to an electrosurgical instrument prepared for performing such method.

One common disease in the prostate is the benign prostatic hyperplasia (BPH). It is initially treated with medications or minimally invasive procedures, such as insertion of a metal anchor also known as UroLift. Such UroLift is a metal anchor that pushes the prostate tissue to the side, enlarging the urethra. However, the effectiveness of UroLift may decrease over time and a transurethral resection (TUR) using a PlasmaCut mode might be necessary. During such transurethral resections, there is a possibility that the electrode of the electrosurgical instrument comes into contact with the UroLift. Such contact can potentially lead to damage to the electrode.

Accordingly, possible damage of bipolar electrodes made of precious metal alloy during the procedure of the transurethral resection might incur. This might lead to increased surgical costs due to the use of multiple electrodes per procedure. A further result may be that there is an uncertainty for the surgeon and potential risks of electrode metal parts remain in the patient after electrode damage.

Accordingly, it is an object of the present invention to overcome at least one of the drawbacks explained above. In particular, there should be a solution proposed according to which it is possible to perform a transurethral resection even when a contact with such metal piece in the region of operation can be expected. However, the solution shall not be restricted to the particular type of operation described above. It is at least an object of the present invention to provide an alternative solution when compared to so far known solutions.

According to the invention, a method is suggested according to claim 1. Such method is thus for controlling an electrosurgical instrument, in particular a resectoscope in transurethral resection, for cutting, vaporizing or thermally treating body tissues. The method is thus directed to the functioning of such electrosurgical instrument. Such electrosurgical instrument has one or two instrument poles. Accordingly, the electrosurgical instrument can be a bipolar electrosurgical instrument or a monopolar electrosurgical instrument.

The electrosurgical instrument is controlled by an RF signal in order to control an electrical instrument current via one instrument pole or over the two instrument poles when the RF signal is activated. Accordingly, the electrosurgical instrument, be it bipolar or monopolar, works in a well-known way using such RF signal to control the electrical instrument current which is used for performing the treatment on the body tissue. I.e., the cutting, vaporizing or thermally treating is done by the electrical instrument current. The RF signal may be an electrical instrument voltage applied to the electrosurgical instrument and resulting in the electrical instrument current. The RF signal may be the electrical instrument current, i.e. the electrical instrument current is provided as the RF signal and may on the other hand result in an electrical instrument voltage.

It is suggested that the method is prepared to identify a contact of one or both instrument poles with an electrically conductive solid, i.e. an electrically conductive piece. In particular, the method is prepared to identify a contact with a metal piece such as the above explained metal anchor, known as UroLift. However, the electrically conductive solid could also be a piece of different material, such as carbon.

The proposed method is based on the idea that such contact with an electrically conductive solid, in particular such metal anchor, cannot be avoided. Instead, it is possible to identify such contact between at least one pole of the instrument and said electrically conductive solid. If such contact is identified, measures can be taken to avoid a damage of the instrument, such as temporally switching off the instrument or changing the operation of the instrument in any other way. Identifying a contact between at least one of the instrument poles and the electrically conductive solid also helps understanding unexpected any reaction or behaviour of the instrument or the operating of it.

The method is prepared to identify said contact and therefore the method can be implemented on the electrosurgical instrument or a generator operating the electrosurgical instrument.

According to one aspect, the method operates such that in order to identify the contact of one or both instrument poles with an electrically conductive solid, the RF signal and/or at least one of the resulting electrical signal resulting from the RF signal be evaluated. The electrosurgical instrument is controlled by an RF signal and this RF signal, for example an RF voltage, may be influenced, i.e. changed, in case of contact of one or both instrument poles with an electrically conductive solid. Such change can be monitored and evaluated. If for example the RF signal is a voltage, such voltage might observe a drop in amplitude upon such contact of an instrument pole with the electrically conductive solid. Of course, the RF signal could also be a current.

If the RF signal is a voltage signal, a current will be a resulting electrical signal. Such resulting electrical signal can also be influenced, i.e. changed, upon contact of at least one instrument pole with the electrically conductive solid. Such change of the resulting electrical signal can also be monitored and evaluated. It is also possible to evaluate a combination of the RF signal and the resulting electrical signal. In this way, a dependency between the RF signal and the resulting electrical signal can be evaluated in order to identify said contact.

Of course, it is also possible that the RF signal is a current signal and the resulting electrical signal is a voltage signal.

According to one aspect, it is suggested that in order to generate the instrument current, the RF signal is applied as an instrument voltage to the one or the two instrument poles, resulting in the instrument current. In order to identify the contact of the one or both instrument poles with an electrically conductive solid, the instrument voltage and the instrument current are evaluated.

Accordingly, it is suggested evaluating both, the instrument voltage and the instrument current. The instrument current is the result of the instrument voltage which is provided as the RF signal. By evaluating both signals, a relationship can be observed and it was found that the relationship of these two signals upon the contact of at least one pole with the electrically conductive solid changes in a way that enables the method to identify such contact. It was understood that such contact with the electrically conductive solid may have such a significant impact on the relationship of these two signals that accordingly such contact can be identified with a very high degree of certainty. It was also found that no additional sensors in the area of the poles at or close to such contact with the electrically conductive solid would be necessary.

According to one aspect, the identification of the contact of the one or both instrument poles with an electrically conductive solid is carried out depending on a characteristic of the instrument current and depending on a characteristic of the instrument voltage.

It was found that not only the amplitude of the instrument voltage as well as the amplitude of instrument current is relevant for identifying such contact with the electrically conductive solid, but evaluating such characteristics of the instrument current and instrument voltage provides good information to identify such contact.

It was realized that such contact of the one pole of a monopolar instrument with the electrically conductive solid or the contact of both instrument poles in case of a bipolar instrument results in a short circuit. Simply speaking, in that case, an instrument voltage may drop and an instrument current may rise. However, depending on the operational phase of the electrosurgical instrument there is also a short circuit but without contact with the electrically conductive solid.

Accordingly, it was found that simply identifying a short circuit might not be enough in order to identify a contact with the electrically conductive solid. But it was also found that such short circuit in case of contact with the electrically conductive solid on the one hand and a regular short circuit without contact to such electrically conductive solid on the other hand leads to different characteristics of at least one of the characteristic of the instrument current and the characteristic of the instrument voltage.

In particular, the evaluation of the characteristic of the instrument current and the characteristic of the instrument voltage gives a clear information whether a regular short circuit without contact to the electrically conductive solid or an exceptional short circuit with contact with an electrically conductive solid occurred. It might be enough to consider only the characteristic of one of the instrument current or instrument voltage. In that case the amplitude of the other might be considered.

Accordingly, it is suggested evaluating both characteristics of the instrument current and the instrument voltage, or at least one of it. The instrument current may be the result of the instrument voltage being the RF signal generated. On the other hand, the RF signal could also be the instrument current and the instrument voltage could be the resulting signal. In both cases, a relationship between the characteristic of the instrument current and the characteristic of the instrument voltage is considered, or a relationship between the characteristic of the one signal and the amplitude and/or RMS value of the other signal.

According to one aspect, the identification of the contact of one or both instrument poles with an electrically conductive solid is carried out depending on at least an amplitude and/or RMS value of the instrument current and/or of the instrument voltage and at least one harmonic analysis of the instrument voltage and/or of the instrument current.

It was found that the contact with the electrically conductive solid has a big impact of harmonics of the RF signal and/or the resulting electrical signal that results from the generated RF signal. For evaluating the harmonics of the instrument voltage and/or instrument current, the amplitude of such harmonics can be compared to the amplitude or RMS value of the instrument current. Or the amplitude of such harmonics can be compared to amplitudes of harmonics of the same signal when there was no contact to any electrically conductive solid.

It was found that at least one of the instrument current and instrument voltage shows at least one significant harmonic in case of contact with the electrically conductive solid. It was particularly found that in case of a short circuit without contact to the electrically conductive solid there are no or significantly less harmonics when compared with the situation of a short circuit in case of a contact with the electrically conductive solid. Accordingly, it is suggested analysing such harmonics. Of course, small harmonics could also occur in case of a short circuit without contact to the electrically conductive solid, but such harmonic is significantly much lower.

In order to make such evaluation, it is suggested considering a significance level for the amplitude of the harmonics. If the amplitude of all harmonics is below such significance level, i.e. there is no harmonics having an amplitude above the significance level, there is no significant harmonics.

In particular, such significance level may be in the order of 5% or less of the amplitude or RMS value of the corresponding instrument current or instrument voltage, respectively. The significance level may in particular be in the range between 1% to 5% of the amplitude or RMS value of the corresponding signal.

According to one aspect, the identification of the contact of the one or both instrument poles with an electrically conductive solid is carried out depending on at least one harmonic of the instrument current and/or of the instrument voltage and wherein in particular the at least one harmonic is compared with at least one reference harmonic and a contact with the electrically conductive solid is only assumed if the amplitude of the harmonic is at least 200% of the amplitude of the at least one reference harmonic.

For this aspect, the same idea occurs as explained above, namely that a significant harmonic identifies a contact with an electrically conductive solid. Therefore, a reference harmonic may be considered. In order to do that, it might be enough to use an amplitude of such reference harmonic. Such amplitude may be stored as reference value and it may be based on empirical values determined in simulations or collected in previous operations of the electrosurgical instrument. The amplitude may also be an RMS-value.

According to one aspect, depending on the instrument current and depending on the instrument voltage, the contact with an electrically conductive solid is identified when an instrument current is identified that has a higher amplitude and/or RMS value than an instrument current in a plasma phase, and at least one harmonic of the instrument voltage and/or instrument current is identified.

Usually, the operation of such electrosurgical instrument, in particular a resectoscope, at start has a gas creation phase. In this gas creation phase, there is gas created in the body tissue while the RF signal is applied. During this gas creation phase, it was found that the instrument current and instrument voltage may both have a sinusoidal shape without or without significant harmonics.

Subsequently, a plasma phase will occur in which - simply speaking - there is an electric arc. In this case, besides a change of the amplitude of the instrument current, harmonics may occur which may be quite significant. In particular, a third, fifth and seventh harmonic may have a significant amplitude which can be 30% of the amplitude of the fundamental wave.

It was further found that in case of a contact with the electrically conductive solid the instrument current may rise again and/or the instrument current and/or instrument voltage may have similar amplitudes and/or RMS values when compared with the gas creation phase. However, during contact with the electrically conductive solid, there are also significant harmonics in at least one of the instrument voltage and instrument current. It is suggested using these particulars for identifying the contact with the electrically conductive solid.

Based on that, it was found and used that the instrument current during contact with the electrically conductive solid is significantly higher than in case of the plasma phase. Accordingly, as a criteria it is suggested evaluating if the instrument current is higher than during the plasma phase, in particular at least by a factor of 2 higher and further in particular at least by the factor of 5 higher and even further in particular at least by the factor of 10 higher than during the plasma phase.

At the same time, it is checked whether there is at least one significant harmonic in at least one of the instrument voltage and the instrument current, as this distinguishes the contact with the electrically conductive solid from the gas creation phase.

In particular, the harmonic has at least one frequency component with an amplitude of at least 10%, and in particular at least 30% of an amplitude of the corresponding fundamental wave. Accordingly, a harmonic component, for example the third harmonic, of the instrument current has at least an amplitude of 10% of the amplitude of the fundamental wave of this instrument current. Instead or in addition, a harmonic component, in particular, the third harmonic, of an instrument voltage has at least an amplitude of 10% of the amplitude of the fundamental wave of the instrument voltage.

In addition or as an alternative criterion, such harmonic is identified with respect to a reference instrument voltage or reference instrument current respectively. It is thus suggested that the harmonic has at least one frequency component with an amplitude of at least 150%, and in particular of at least 200% of the same frequency component of a reference instrument voltage and/or of a reference instrument current respectively taken at the same RF signal when no contact with the electrically conductive solid is present.

Accordingly, it is checked whether the particular harmonic, e.g. the third harmonic, or fifth harmonic, or seventh harmonic, is significantly higher than the same frequency component, i.e. the third, fifth or seventh harmonic, respectively, of a reference instrument voltage, if the harmonic of the signal to be checked is also of the instrument voltage.

In the same manner, also a harmonic of the instrument current can be compared with the corresponding harmonic of the reference instrument current.

The reference instrument voltage or the reference instrument current, respectively, are taken at the same RF signal when no contact with the electrically conductive solid is present. In this way, instrument current and/or instrument voltage of the same situation can be compared, whereas only one is for the situation with contact to the electrically conductive solid and the other one is for the situation without the contact to the electrically conductive solid.

According to one aspect, it is suggested that to identify the contact of the one or both instrument poles with an electrically conductive solid at least one gas creation phase, one plasma phase and a metal contact phase can be identified. These three phases have already been explained above. The metal contact phase represents a phase in which the one or both instrument poles have contact with the electrically conductive solid. Accordingly, even though the phase is called metal contact phase, the electrically conductive solid may be metal or may be a material that is electrically conductive but not metal such as carbon or other artificial materials.

The gas creation phase is identified when an essentially sinusoidal instrument current has been detected with an initial RMS value and a substantially sinusoidal instrument voltage has been detected. The plasma phase is identified when an oscillating, non-sinusoidal instrument current with an RMS value less than the RMS value of the instrument current in the gas creation phase has been detected and a sinusoidal instrument voltage with harmonics has been detected.

The metal contact phase is identified when an instrument current is at least two times higher than in the plasma phase and the instrument voltage has at least one harmonic that is at least two times higher than in the gas creation phase. This is suggested when the RF signal is generated as an instrument voltage.

Alternatively, when the RF signal is generated as an instrument current, the metal contact phase is identified when the instrument voltage is at least 50% lower than in the plasma phase, and the instrument current has at least one harmonic that is at least 50% higher than in the gas creation phase.

The metal contact phase is thus identified when an instrument current has an amplitude and/or an RMS value which is higher than in the plasma phase. In particular that is the case when it is at least ten times higher than the amplitude or the RMS value of the instrument current of the plasma phase, and/or if there is an instrument current with an amplitude and/or RMS value within a range of 50% to 150% of the RMS value of the instrument current of the gas creation phase. At the same time it is evaluated if for the instrument voltage at least one harmonic has been detected, in particular at least one harmonic with an RMS value of at least 10% and in particular of at least 50% of an RMS value of the fundamental wave, and/or with an RMS value of at least 150% and in particular of at least 200% of an RMS value of a reference harmonic. The reference harmonic can be defined as explained above. This is suggested when the RF signal is generated as an instrument voltage.

Alternatively, when the RF signal is generated as an instrument current, the metal contact phase is identified when the instrument voltage has an amplitude and/or an RMS value which is lower, in particular by at least 80% lower than the instrument voltage of the plasma phase. At the same time it is evaluated if for the instrument current, at least one harmonic has been detected, in particular at least one harmonic with an RMS value of at least 10% and in particular at least 30% of an RMS value of the fundamental wave, and/or at least one harmonic with an RMS value of at least 150% and in particular at least 200% of a reference harmonic.

Accordingly, the knowledge of said three phases is used to define said criteria. The criteria can be based on the amplitude of the instrument current or the amplitude of the instrument voltage.

In case of using the instrument current, it is checked whether its amplitude is significantly higher when compared with the instrument current of the plasma phase. This is due to the fact that the contact with the electrically conductive solid basically results in a short circuit current and accordingly this instrument current is a kind of short circuit current and has a significantly higher amplitude than during the plasma phase.

However, in particular when the RF signal is given by an instrument current and the instrument voltage is the resulting signal, the instrument voltage would basically collapse in case of contact with the electrically conductive solid. Accordingly, in this case it is checked whether the instrument voltage has a significantly reduced amplitude and that it is in particular the case when the amplitude is by at least 80% lower than the instrument voltage of the plasma phase. In other words, it is at most 20% of the instrument voltage of the plasma phase.

As a second criterion, in order to distinguish the metal contact phase from the gas creation phase, the signals are checked for harmonics. If there are harmonics that have a significant amplitude, it can be assumed that there is contact to the electrically conductive solid. Otherwise, it can be assumed that the gas creation phase is present.

For the second criterion, harmonics of the voltage signal can be checked if the instrument voltage is provided as the RF signal and if it was checked that the instrument current increases significantly when compared to the plasma phase. If, on the other hand, the instrument current is provided as the RF signal and if it was checked that the instrument voltage dropped when compared to the plasma phase, harmonics of the instrument current can be checked.

In any case, harmonics, at least one harmonic, can be checked in two different ways. One possibility is to compare the amplitude of the at least one harmonic with the amplitude of the fundamental wave. The other possibility is to compare the amplitude of the at least one harmonic with the reference harmonic. The reference harmonic is corresponds to the gas creation phase and may be determined offline and implemented as a fixed value, or it may be recorded during the gas creation phase.

According to one aspect, the electrosurgical instrument is controlled by an inverter and the inverter is prepared to specify an instrument voltage with a signal frequency in the range of 200-800 kHz, in particular, 350-450 kHz. Optionally, the inverter is prepared to specify the signal frequency with an accuracy of at least 5 Hz, in particular at least 2 Hz.

It was found that using such inverter that has such a high accuracy with respect to the generated frequency, facilitates the identification of harmonics. In particular, such harmonics are identified using a Fourier transform and that needs a fundamental frequency for calculation. Such fundamental frequency does not need to be identified if it can be given by the inverter with a high accuracy.

According to one aspect, it is suggested that in order to identify the contact of one or both instrument poles with an electrically conductive solid, a frequency analysis, in particular a Fourier analysis of an instrument voltage and/or the instrument current is carried out. In particular, the frequency analysis is performed based on a fundamental frequency and a frequency setpoint for the instrument voltage is used as the fundamental frequency. By using such frequency analysis, in particular the Fourier analysis, higher harmonics can be identified in a reliable way and the identified harmonics, or that it is identified that there are no significant harmonics, can be used to evaluate whether a contact of one or both instrument poles with an electrically conductive solid appears, as explained above. Such frequency analysis can be carried out based on the instrument voltage and/or based on the instrument current. It was realized that harmonics in the instrument voltage as well as harmonics in the instrument current, be it alone or in combination, can be used to identify said contact with an electrically conductive solid.

Using the fundamental frequency as a basis for performing the frequency analysis, in particular for performing the Fourier analysis, facilitates such analysis. In particular, it avoids to identify the relevant frequency. Such fundamental frequency can be received from a controller controlling the RF signal. The controller for controlling the RF signal may control the RF signal based on a set value for the frequency of the RF signal and such set value can be used as the fundamental frequency at least when a high accuracy for providing the RF signal with a frequency according to the frequency setpoint is ensured. Accordingly, the frequency setpoint for the instrument voltage is the setpoint for the frequency of the RF signal.

According to one aspect, the RF signal, i.e. the voltage and/or the instrument current is interrupted when the contact of one or both instrument poles with an electrically conductive solid has been identified. In this way, it is possible to use the electrosurgical instrument in a regular way, even when a contact with an electrically conductive solid may be expected. The methods according to aspects explained above may automatically and quickly identify if such unwanted contact to an electrically conductive solid occurs. In this case, the RF signal may be interrupted avoiding or at least reducing any damage of the one or both electrodes.

Once such contact has been identified and the RF signal being interrupted, shortly after, the use of the electrosurgical instrument can be continued, of course after at least a slight movement, in particular backwards, of the electrosurgical instrument such that the contact with the electrically conductive solid has been terminated.

According to one aspect, it is suggested that if the contact of one or both instrument poles with an electrically conductive solid has been identified, and in particular the RF signal i.e. the voltage and/or the instrument current, has been interrupted, an indication signal is issued indicating to an operator of the electrosurgical instrument, in particular to a surgeon, the contact with an electrically conductive solid. This way the surgeon immediately gets the information of such contact.

In particular the indication signal is or includes an acoustic signal and/or a visual signal, in particular a signal on a screen of the generator or on a device controlling the generator and/or on the electrosurgical instrument. By such acoustic signal the surgeon can immediately get such information even when not looking on any display. By providing such signal on a display, a more precise information of the event may be given.

According to one aspect, it is suggested that after an interruption of the RF signal, i.e. the voltage and/or the instrument current, due to the identification of the contact with an electrically conductive solid, a confirmation input from an operator is requested to restart the RF signal, i.e. to restart the generating of the RF signal.

Accordingly, the electrosurgical instrument for which the method is implemented is prepared to identify said contact with an electrically conductive solid, interrupt the RF signal i.e. the instrument voltage and/or the instrument current, accordingly, and let the operation of the electrosurgical instrument restart again in a simple manner by an operator, in particular a surgeon. This way the operator realizes that there was said contact with the electrically conductive solid leading to the interruption and the instrument enables the operator to react on that by finding a new position, not being in contact with the electrically conductive solid, and then restart the operation.

In addition or alternatively, it is suggested that after or with interruption of the RF signal due to the identification of the contact with an electrically conductive solid, a visual marking is placed on a screen. Such visual marking can in particular be placed on an endoscopic image, showing a distal end of the one or both instrument poles and the point of the distal end when the contact was identified.

Accordingly, the point of the distal end of the one or both instrument poles when the contact was identified is thus localizing the contact point with the electrically conductive solid. Such screen, in particular, an endoscopic image, can be used to assist an operator using the electrosurgical instrument. When the contact with the electrically conductive solid is identified, the position of the distal end of the one or both electrodes is thus the position of the electrically conductive solid, at least the point of contact with it.

It is thus suggested marking this point and thus guiding the operator to avoid this position with the one or both electrodes. Such point can be permanently marked at least for a regular duration of an operation and in case there is a further contact with the electrically conductive solid identified, a further marker can be placed identifying this second contact. This may assist the operator to navigate between such contact positions.

Preferably, each time the RF signal current is interrupted, thus avoiding a damage of the electrosurgical instrument. In this way, it could be possible to accept a plurality of contacts and in that case each contact would result in such marker. Accordingly, a plurality of such identified contact points could even build up a complete image of the UroLifts or other piece forming the electrically conductive solid placed in the area of operation.

According to one aspect, the method is designed such that an interruption of the RF signal after the identification of a contact of the one or both instrument poles with an electrically conductive solid takes place within less than 50 periods of the fundamental frequency of the RF signal. In particular, it takes place within five periods or even within one period or a half wave of the RF signal, i.e., within half a period of the RF signal.

It was found that such quick interruption avoids damage to the electrodes. It was also found that using a Fourier transform can get information of the characteristics of the RF signal for one period of the RF signal, in the meaning of one period of the fundamental frequency of the RF signal. Accordingly, the interruption can also be triggered within that time.

It was even found that a frequency analysis and even a Fourier transform is possible even for just a half wave, as such half wave already comprises all necessary information needed for such Fourier transform or other frequency analysis. If the interruption is done within one period or even within one half wave, there is almost no damage of the electrode poles to be expected.

However, it was found that depending on the generator for providing the RF signal, such quick frequency analysis within one period or even half a period can be performed but other inverters might not have enough processor capacity to perform such quick frequency analysis but could at least identify the contact with an electrically conductive solid within 50 periods of the fundamental frequency. For that situation it was found that even an interruption within 50 periods of the fundamental frequency after contact with the electrically conductive solid is quick enough to avoid any severe damage on the electrode poles.

According to one aspect, an electrosurgical generator is suggested for controlling an electrosurgical instrument, in particular a resectoscope in transurethral resection, for cutting, vaporizing or thermally treating body tissue, wherein the electrosurgical instrument has one or two instrument poles and the electrosurgical generator is adapted to control the electrosurgical instrument by an RF signal in order to control an electrical instrument current via one instrument pole or via the two instrument poles when the RF signal is activated, wherein the electrosurgical generator is prepared to identify a contact of one or both instrument poles with an electrically conductive solid.

The electrosurgical generator may comprise an inverter in order to provide the RF signal. In this way, the electrosurgical generator is adapted to execute the control of the electrosurgical instrument. The electrosurgical generator may also have sensors for the RF signal, and/or for measuring the instrument current and/or for measuring an instrument voltage. Such sensors may be connected to an internal controller of the electrosurgical generator. Accordingly, the electrosurgical generator comprises such controller and can use this controller to identify said contact of one or both instrument poles with an electrically conductive solid. This can be done based on measured or estimated instrument current and/or instrument voltage.

According to one aspect, the electrosurgical generator comprises a generator controller for controlling the RF signal and for identifying the contact of one or both instrument poles with an electrically conductive solid. Therefore, such generator controller can be programmed accordingly and can in particular control an inverter for providing the RF signal.

The electrosurgical generator, in particular the generator controller, may be prepared to execute a method according to any of the preceding explained aspects of the explained method. In this way, an electrosurgical generator can operate as explained with respect to the method aspects and can thus achieve the explained advantages.

The invention will now be explained in more detail by way of example based on the attached figures.
- **Figure 1**: shows an electrosurgical generator with an attached electrosurgical instrument in a schematical view.
- **Figure 2**: shows a prostate with implemented UroLifts and in inserted resectoscope in a schematical view.
- **Figures 3A - 3C**: show an instrument voltage and an instrument current of an electrosurgical instrument and a corresponding frequency spectrum during different phases.

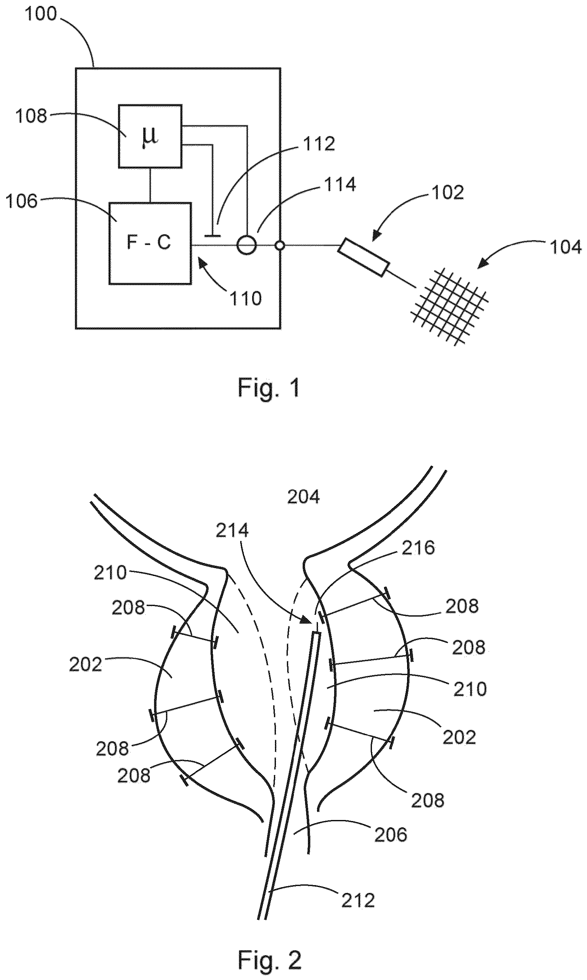

**Figure 1** shows an electrosurgical generator 100 to which an electrosurgical instrument 102 is connected operating on a tissue 104, i.e. a working element, just schematically illustrated. The tissue 104 can be a prostate or part of it.

The electrosurgical generator 100 comprises a frequency converter 106 which is controlled by a control device 108. The frequency converter 106 when in operation provides at its output 110 a voltage signal as an RF signal which is applied to the attached electrosurgical instrument 102, resulting in an operational current.

There is a voltage sensor 112 for measuring the voltage signal and a current sensor 114 for measuring the operational current. These sensors 112, 114 are connected to the control device 108 for further consideration.

The control device 108 calculates from the voltage sample values provided by the voltage sensor 112 and current sample values provided by the current sensor 114 amplitude or RMS values and from at least one of the voltage and current samples harmonics using a Fourier transform. Based on that, the control device 108 identifies any contract with the instrument 102 with an electrically solid, interrupts the generation of the RF signal and indicates such contact to the user, i.e. operator of surgeon.

The voltage sensor 112 might not be necessary as it is known what kind of voltage the voltage converter 106 generates. It is also possible that the voltage sensor 112 is connected to the frequency converter 106 directly. It is also possible that the control device 108 is part of the frequency converter 106.

**Figure 2** shows a prostate 202 located between a bladder 204 and the Urethra 206. When the prostate expands, what often happens for men getting older that could lead to problems when urinating. In order to stop or reduce such extension of the prostate 202 so called UroLifts 208 can be applied. Such UroLifts 208 are often formed as metal anchors and thus made of metal.

However after some years it is possible that the prostate 202 further expands by having tissue 210 grow inside of the prostate. Such further tissue 210 is indicated by the dashed lines. In order to remove such tissue 210 a medical operation using an electrosurgical instrument, in particular a resectoscope 212 may be necessary. In figure 2 only part of the resectoscope is shown and the resectoscope 212 may have a distal end 214. At the distal end 214 of the resectoscope 212 there is an electrode 216 providing an instrument current that flows from the electrode 216 into the tissue 210. In a different embodiment the distal end 214 of the resectoscope may have 2 electrodes wherein an instrument current flows from one electrode to the other electrode.

However when using the resectoscope 212 having an instrument current flow into the tissue or to another electrode, the distal end 214 and thus the electrode 216 may get in contact with one of the UroLifts 208. This situation is shown in figure 2 such that the distal end 214 is close to one of the UroLifts 208 and is about to have contact with one of this UroLifts 208.

The UroLifts are usually made of metal but could also be another material that is electrically conductive. Accordingly one piece of such UroLift is an electrically conductive solid.

During operating with the resectoscope 212 in order to remove at least part of the shown additional tissue 210 the electrode 216 may get in contact with one of such UroLift 208 and that could cause a damage to electrode 216. In that case the instrument current may flow into one of the UroLifts 208 causing said damage.

To avoid such damages a contact with the electrical conductive solid is identified and the instrument current can be interrupted immediately. Such interruption can be shown to the person operating the resectoscope 212 and accordingly it is possible to pull back the resectoscope 212 and thus the distal end 214 and electrode 216 just a little bit and then to continue the operation of removing part of the tissue 210 in some distance of the contacted UroLift 208.

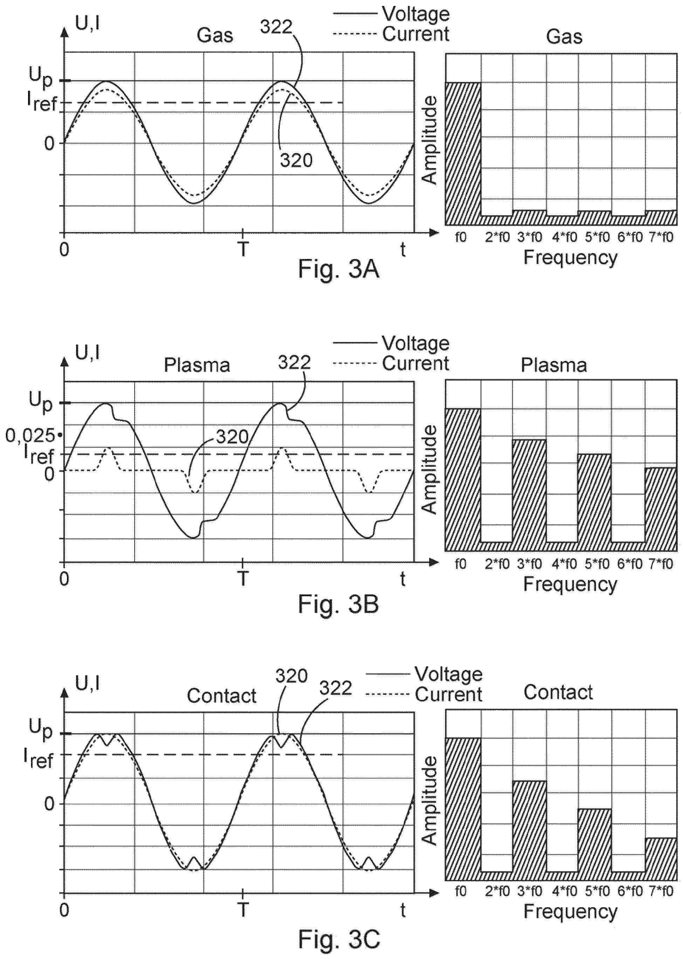

**Figures 3A to 3C** show instrument voltages and instrument currents over time and a corresponding frequency spectrum in different situations, i.e. different phases when operating the electrosurgical instrument. A fundamental frequency is the same for all diagrams. The abscissa, i.e. the time-axis, of all three Figures shows the time-values by way of reference to the oscillation period T of the fundamental frequency f₀.

Figures 3A and 3B are related to a typical cutting operation with an electrosurgical instrument, in particular a resectoscope. Figure 3C is related to a situation when during such operation an electrosurgical instrument, i.e. one or two electrodes have contact with a metal piece or other electrically conductive solid.

Accordingly figure 3A is related to a gas creation phase. Such gas creation phase can be understood as a first state of operating the resectoscope. During such gas creation phase there is a high instrument current which forms an electrical insulating gas layer around the electrode. The instrument current during the gas creation phase is high because the electrode is fully covered by conductive saline solution with a low impedance. Such impedance can be around 25 Ohm to 50 Ohm. It was found that as in this phase there is only ohmic current flow to be expected, there shall be no harmonics. It is to be mentioned that there is no sharp boundary from one phase to the other but a smooth transition in which current and voltage find their form.

This situation is illustrated in figure 3A and accordingly there is an instrument current 320 and an instrument voltage 322. The amplitude in the meaning of an RMS value may be at a level of a reference current I_{ref}. The amplitude of the instrument voltage 322 does not change very much within the different phases and thus within the different diagrams according to figure 3A to figure 3C. Accordingly there is just a peak voltage U_{P} indicated as a generalized indication of the amplitude.

As can be seen in the diagram of figure 3A the instrument current 320 and the instrument voltage 322 are basically sinusoidal without harmonics. This is further illustrated in the frequency spectrum also shown in figure 3A which only comprises one significant value for the fundamental frequency, indicated as f₀.

Figure 3B also shows the instrument current 320 and instrument voltage 322. The characteristics are different when compared to figure 3A and 3C, but the same reference numerals are used in all three figures. Figure 3B thus shows the plasma phase as the second state. In this state the electric field across the gas layer causes ionization and plasma breakthroughs. The instrument current during the plasma phase is low because the electrode is fully covered by an insulating gas layer. Current flow occurs due to ionization and breakthroughs which let some harmonics occur in the frequency spectrum.

Accordingly figure 3B shows an amplitude of the instrument current 320 of 0,025 of the level of the reference current I_{ref} of Figure 3B. Accordingly the amplitude of the instrument current during the gas creation phase as shown in figure 3A is, in this example, 40 times as high as during the plasma phase shown in figure 3B. Obviously the time diagram of Figure 3B has a different scale for the amplitude of the current than the time diagrams of Figures 3A and 3C.

Harmonics are present in the instrument current 320 as well as in the instrument voltage 322. The frequency spectrum for the instrument voltage 322 in figure 3B thus shows values at the fundamental frequency f₀ and also significant values at the third, fifth and seventh harmonic, i.e. at 3*f₀, 5*f₀ and 7*f₀.

It was found that a subsequent metal contact, or contact to any other electrically conductive solid can be detected taking the RMS value of the current and the availability or occurrence of harmonics into account. Accordingly the idea is to implement a rapid detection of metal contact (or contact of any other electrically conductive solid) utilizing electrical RF characteristics and implement a subsequent RF shutdown to prevent damage to the electrode.

This is based on realizing that the instrument current during a metal contact is high because the metal probably disturb the insulation gas layer and adds an ohmic current path between electrode and saline solution. The harmonics probably occur due to plasma breakthroughs at the remaining parts of the electrode. Accordingly this was understood and is used for identifying such contact with an electrical conductive solid.

Figure 3C thus shows the instrument current 320 and instrument voltage 322 during metal contact of the electrode or contact with any other electrically conductive solid. As can be seen the amplitude of the instrument current 320 and also the amplitude of the instrument voltage 322 are similar when compared to the gas creation phase as shown in figure 3A. However the instrument voltage 322 comprises harmonics and this way it can be distinguished between the gas creation phase according to figure 3A and the phase of metal contact according to figure 3C. To further emphasize this, figure 3C also shows a frequency spectrum of the instrument voltage 322 having values at the fundamental frequency as well as for the third, fifth and seventh harmonics. The frequency spectrum of figure 3C looks similar to figure 3B but is not necessarily identical.

Based on that the following characteristics could be found and form the basis for identifying the contact with the electrically conductive solid. These characteristics are thus simplified in the following table. Given values just have the purpose of illustration.

| | RMS current | Harmonics | Reference |
|---|---|---|---|
| Gas creation (normal) | High (e.g. > 1 A | No | Figure 3A |
| Plasma (normal) | Low (e.g. < 1 A) | Yes | Figure 3B |
| Metal contact (abnormal) | High (> 1 A) | Yes | Figure 3C |

The gas creation phase and the plasma phase are thus normal phases and the metal phase according to figure 3C, briefly depicted as metal contact, is an abnormal situation and can be identified by a high instrument current with at the same time the presence of harmonics.

To verify this understanding tests were made with the following results.

The lifetime of electrodes with its actual platinum-iridium alloy when touching metal was tested to be around 20 ms. The detection of high currents and harmonics can be done in maximum 10 periods (28.5 µs), subsequent RF shutdown can be achieved within 1 µs. The extremely short duration of approximately 30 µs allows for detection of metal contact and rapid RF shutdown, effectively preventing any damage to the electrode.

Figures 3A to 3C shows situations were a RF signal is generated as an instrument voltage with resulting instrument current as shown in these figures 3A to 3C. However the inventive idea can also be applied to systems where the instrument current is given and the instrument voltage results. Corresponding diagrams will of course be different but a contact with a different electrically conductive solid can then be identified by a low instrument voltage being significantly lower than during the plasma phase and at the same time having harmonics in the voltage signal.

## Claims

1. Method for controlling an electrosurgical instrument, in particular a resectoscope in transurethral resection, for cutting, vaporizing or thermally treating body tissues, wherein:
- the electrosurgical instrument has one or two instrument poles , and
- the electrosurgical instrument is controlled by an RF signal in order to control an electrical instrument current via one instrument pole or via the two instrument poles when the RF signal is activated, wherein
- the method is prepared to identify a contact of one or both instrument poles with an electrically conductive solid.

2. The method according to claim 1, **characterized in that**
- to identify the contact of one or both instrument poles with an electrically conductive solid
- the RF signal, and/or
- at least one of the resulting electrical signal resulting from the RF signal
- be evaluated.

3. The method according to claim 1 or 2, **characterized in that**
- to generate the instrument current, the RF signal is applied as an instrument voltage to the one or the two instrument poles, resulting in the instrument current, and
- to identify the contact of the one or both instrument poles with an electrically conductive solid, the instrument voltage and the instrument current are evaluated.

4. Method according to any of the preceding claims, **characterized in that**
- the identification of the contact of the one or both instrument poles with an electrically conductive solid is carried out depending on a characteristic of the instrument current and/or a characteristic of a or the instrument voltage.

5. Method according to any of the preceding claims, **characterized in that**
- the identification of the contact of the one or both instrument poles with an electrically conductive solid is carried out depending on at least
- an amplitude and/or rms value of the instrument current and/or of the instrument voltage and
- at least one harmonic analysis of the instrument voltage and/or of the instrument current.

6. Method according to any of the preceding claims, **characterized in that**
the identification of the contact of the one or both instrument poles with an electrically conductive solid is carried out depending on
- at least one harmonic of the instrument current and/or instrument voltage and wherein in particular
- the at least one harmonic is compared with at least one reference harmonic and a contact with the electrically conductive solid is only assumed if the amplitude of the harmonic is at least 200% of the amplitude of the at least one reference harmonic.

7. Method according to any of the preceding claims, **characterized in that**
- depending on the instrument current and depending on a or the instrument voltage, the contact with an electrically conductive solid is identified when
- an instrument current is identified that has a higher amplitude and/or a higher RMS value than an instrument current in a plasma phase in which the instrument current occurs as a plasma current, in particular by a factor of at least 2 higher and further in particular at least by a factor of 5 higher, and
- at least one harmonic of the instrument voltage and/or instrument current is identified, and in particular the harmonic
- has at least one frequency component with an amplitude of at least 10%, and in particular at least 30% of an amplitude of the corresponding fundamental wave and/or
- has at least one frequency component with an amplitude of at least 150%, and in particular at least 200% of the same frequency component of a reference instrument voltage and/or instrument current taken at the same RF signal when no contact with the electrically conductive solid is present.

8. Method according to any of the preceding claims, **characterized in that**
to identify the contact of the one or both instrument poles with an electrically conductive solid, at least one gas generation phase, one or the plasma phase and a metal contact phase, representing a phase in which the one or both instrument poles have contact with an electrically conductive solid, can be identified, where
- the gas creation phase is identified when an essentially sinusoidal instrument current has been detected having an initial RMS value, and when a substantially sinusoidal instrument voltage has been detected,
- the plasma phase is identified when an oscillating, non-sinusoidal instrument current with an RMS value less than the RMS value of the instrument current in the gas generation phase has been detected and when a sinusoidal instrument voltage with harmonics has been detected, and
- the metal contact phase is identified when
- an instrument current is at least two times higher than in the plasma phase and the instrument voltage has at least one harmonic that is at least two times higher than in the gas creation phase or
- when the instrument voltage is at least 50% lower than in the plasma phase, and the instrument current has at least one harmonic that is at least 50% higher than in the gas creation phase.

9. Method according to any of the preceding claims, **characterized in that**
- the electrosurgical instrument is controlled by an inverter, and
- the inverter is prepared to specify an instrument voltage with a signal frequency in the range of 200-800 kHz, in particular 350-450kHz, and in particular:
- the inverter is prepared to specify the signal frequency with an accuracy of at least 5 Hz, in particular at least 2 Hz.

10. Method according to any of the foregoing claims, **characterized in that**
- in order to identify the contact of one or both instrument poles with an electrically conductive solid, a frequency analysis, in particular a Fourier analysis, of an instrument voltage and/or the instrument current, is carried out, and in particular
- the frequency analysis is performed based on a fundamental frequency, and
- a frequency setpoint for the instrument voltage is used as the fundamental frequency.

11. Method according to any of the foregoing claims, **characterized in that**
- the RF signal is interrupted when the contact of one or both instrument poles with an electrically conductive solid has been identified.

12. Method according to any of the preceding claims, **characterized in that**
- if the contact of one or both instrument poles with an electrically conductive solid has been identified, and in particular the RF signal has been interrupted, an indication signal is issued indicating to an operator of the electrosurgical instrument the contact with an electrically conductive solid, wherein in particular the indication signal is or includes
- an acoustic signal and/or
- a visual signal, in particular a signal on a screen of the generator or on a device controlling the generator and/or on the electrosurgical instrument.

13. Method according any of the preceding claims, **characterized in that**:
- after an interruption of the RF signal due to the identification of the contact with an electrically conductive solid, a confirmation input from an operator is requested to restart the RF signal, and/or
- after or with interruption of the RF signal due to the identification of the contact with an electrically conductive solid, a visual marking is placed on a screen, in particular in an endoscopic image, showing a distal end of the one or both instrument poles and the point of the distal end when the contact was identified, and/or
- an interruption of the RF signal after the identification of a contact of the one or both instrument poles with an electrically conductive solid takes place within less than 50 periods of a or the fundamental frequency of the RF signal, in particular it takes place within five periods, in particular within one period or a half-wave of the RF signal.

14. Electrosurgical generator for controlling an electrosurgical instrument, in particular a resectoscope in transurethral resection, for cutting, vaporizing or thermally treating body tissues, wherein:
- the electrosurgical instrument has one or two instrument poles , and
- the electrosurgical generator is adapted to control the electrosurgical instrument by an RF signal in order to control an electrical instrument current via one instrument pole or via the two instrument poles when the RF signal is activated, wherein
- the electrosurgical generator is prepared to identify a contact of one or both instrument poles with an electrically conductive solid.

15. Electrosurgical generator according to claim 14, **characterized in that**
- the electrosurgical generator comprises a generator controller for controlling the RF signal and for identifying the contact of one or both instrument poles with an electrically conductive sold, and/or
- the electrosurgical generator is prepared to execute a method according to any of claims 1 to 13, in particular using the generator controller.
